# EUROPEAN PATENT SPECIFICATION

(11) **EP 1 910 835 B1**
(45) Date of publication and mention of the grant of the patent: **07.03.2012**
(21) Application number: 06777834.0
(22) Date of filing: 18.07.2006
(51) Int. Cl.: G01N 33/569, G01N 33/551

(54) **SUPPORT HAVING NANOSTRUCTURED TITANIUM DIOXIDE FILM AND USES THEREOF**
TRÄGER MIT NANOSTRUKTURIERTEM TITANDIOXIDFILM UND VERWENDUNGEN DAVON
SUPPORT POURVU D'UN FILM DE DIOXYDE DE TITANE NANOSTRUCTURE ET UTILISATIONS DE CELUI-CI

(30) Priority: 21.07.2005 EP 05015869
(43) Date of publication of application: 16.04.2008
(73) Proprietor: Tethis S.p.A., 20123 Milano (IT)
(72) Inventor: CARBONE, Roberta, Tethis S.r.l., 20121 Milano (IT); PELICCI, Pier Giuseppe, Instituto Europeo di Oncologia, 20141 Milano (IT); PISERI, Paolo, Tethis S.r.l., 20121 Milano (IT); MILANI, Paolo, Tethis S.r.l., 20121 Milano (IT); BARBORINI, Emanuele, Tethis S.r.l., 20121 Milano (IT); BONGIORNO, Gero Antonio, Univerità di Milano, 20133 Milano (IT)
(74) Representative: Schnabel, Jörg
(86) International application number: PCT/EP2006/064377
(87) International publication number: WO 2007/009994

(56) References cited:
- WO-A-2004/011672
- FURUZONO T ET AL: "PHOTOREACTIVITY AND CELL ADHESIVENESS OF AMINO-GROUP-MODIFIED TITANIUM DIOXIDE NANO-PARTICLES ON SILICONE SUBSTRATE COATED BY COVALENT LINKAGE" JOURNAL OF MATERIALS SCIENCE LETTERS, CHAPMAN AND HALL LTD. LONDON, GB, vol. 22, no. 24, 15 December 2003 (2003-12-15), pages 1737-1740, XP001199293 ISSN: 0261-8028
- PAUNESKU TATJANA ET AL: "Biology of TiO2-oligonucleotide nanocomposites" NATURE MATERIALS, NATURE PUBLISHING GROUP, LONDON, GB, vol. 2, no. 5, May 2003 (2003-05), pages 343-346, XP002373955 ISSN: 1476-4660
- PAUNESKU, T., ET AL: "Intracellular localization of titanium dioxide-biomolecule nanocomposites" J.PHYS IV FRANCE, vol. 104, 2003, pages 317-319, XP001247559
- BARBORINI E ET AL: "Supersonic cluster beam deposition of nanostructured titania" EUROPEAN PHYSICAL JOURNAL D: ATOMS, MOLECULES, CLUSTERS AND OPTICAL PHYSICS, EDP SCIENCES, LES ULIS,, FR, vol. 24, 2003, pages 277-282, XP002315139 ISSN: 1434-6060
- TOPOGLIDIS, E. ET AL: "Protein Adsoprtion on Nanocrystalline TiO2 Films: An Immobilization Strategy for Bioanalytical Devices" ANALYTICAL CHEMISTRY, vol. 70, no. 23, 1 December 1998 (1998-12-01), pages 5111-5113, XP002355943
- BARBORINI E ET AL: "Engineering the nanocrystalline structure of TiO2 films by aerodynamically filtered cluster deposition" APPLIED PHYSICS LETTERS, AIP, AMERICAN INSTITUTE OF PHYSICS, MELVILLE, NY, US, vol. 81, no. 16, 14 October 2002 (2002-10-14), pages 3052-3054, XP012032220 ISSN: 0003-6951
- CHUN-PING JEN ET AL: "Nanoparticle-mediated in-vitro gene transfection on the micro flectroporation chip" TRANSDUCERS, SOLID-STATE SENSORS, ACTUATORS AND MICROSYSTEMS, 12TH INNATIONAL CONFERENCE ON, 2003, PISCATAWAY, NJ, USA,IEEE, vol. 2, 9 June 2003 (2003-06-09), pages 1047-1050, XP010647809 ISBN: 0-7803-7731-1
- HUGHES, C., ET AL: "Streptavidin Paramagnetic Particles Provide a Choice of Three Affinity-Based Capture and Magnetic Concentration Strategies for Retroviral Vectors" MOLECULAR THERAPY, vol. 3, no. 4, April 2001 (2001-04), pages 623-630, XP002403412
- PRABHA, S. AND LABHASETWAR, V.: "Nanoparticle-Mediated Wild-Type p53 Gene Delivery Results in Sustained Antiproliferative Activity in Breast Cancer Cells" MOLECULAR PHARMACEUTICALS, vol. 1, no. 3, 2004, pages 211-219, XP002403413
- ASAHARA T ET AL: "STEM CELL THERAPY AND GENE TRANSFER FOR REGENERATION" GENE THERAPY, MACMILLAN PRESS LTD., BASINGSTOKE, GB, vol. 7, no. 6, March 2000 (2000-03), pages 451-457, XP000916745 ISSN: 0969-7128

## Description

The present invention relates to supports for bioassays and the use thereof in cell culturing and in cell-based methods and assays. More precisely, the invention provides solid materials coated with films of nanostructured titanium dioxide suitable for the immobilisation of viruses and for cell-adhesion. The nanostructured TiO₂ film-coated support of the invention is particularly useful for the preparation of microarrays for genetic and phenotypic analysis.

### Background of the invention

Since the genome has been completely sequenced the need of exploring the full repertoire of proteins for their function in the normal and pathological conditions has become a main goal for new drug target identification and gene therapy applications [1].

The high throughput analysis for gene function studies requires high efficiency of gene transduction and the possibility to analyze different cellular model systems, in a convenient format, on a suitable support, possibly a slide, where thousands of genes can be analyzed simultaneously by simple methods like immunofluorescence.

Several methods of gene transduction have been proposed including uptake of plasmid DNA by transfection [2], electroporation [3], microinjection [4], and viral infection [5].

The most efficient among these technologies is the virus-mediated gene delivery since different kinds of cellular systems, primary and cancer cells of mammalian origin, have shown to be successfully transduced with different viral vectors.

Titanium dioxide (Ti0₂) is known as a biocompatible material [6] and it is widely used in implants. Protein and cell attachment mechanisms on TiO₂ films have been studied [7, 8]. The adsorption of proteins on nanocrystalline TiO₂ films has been studied in [19]. The modification of the surface at the nanoscale has been recognized as important to favour cell adhesion, however the mechanisms influencing the cell attachment on a nanostructured substrate are largely unknown [9].

TiO₂-Oligonucleotide nanocomposites have recently been proposed as vectors for the introduction into cells of genetic material [10]. These nanocomposites retain the bioactivity of the oligonucleotide DNA and they can be photoactivated to induce nucleic acid endonuclease in view of gene therapy.

The realization of a viral array, where each cluster of cells will be infected by substrate-immobilized viral particles is still a challenge: the method should allow a) viral immobilization on the substrate while maintaining virus activity toward target cells b) the virus should be immobilized but be able to enter target cells c) the substrate of immobilization should be biocompatible to permit cell attachment, infection and proliferation and also be optically transparent.

The technical problem underlying the present invention is therefore to provide novel supports for use in bioassays using virus and/or cells.

The solution to the above technical problem is provided by the embodiments of the present invention as characterized in the claims.

### Description of the invention

In particular, it has been found that nanostructured TiO₂ obtained by deposition of nanoparticles from the gas phase provides a valuable substrate for virus adsorption and cell-adhesion, entirely compatible with cell culture and growth.

According to a first aspect, the invention is directed to a solid support especially suitable for *in vitro* bioassays, consisting of a biocompatible substrate material coated at least partially with a nanostructured TiO₂ film having viruses and/or cells immobilised on the surface thereof.

Any material suitable for cell or tissue culturing and for cell-based assays can be used as a substrate for TiO₂ film coating, preferably glass, plastic, ceramic, metal or a biodegradable or undegradable biopolymeric materials. As conventionally used, the term "biocompatible" indicates that the material should not affect or interfere with normal cell activities, e.g. *in vitro* growth and proliferation, nor interact with, or alter, the substances used in the preparation of cell cultures.

The support material may be differently shaped depending on the application sought and on the assay format. Suitable supports include, but are not limited to, slides, e.g. microscope slides, dishes, flasks or plates (such as microtiter plates having multiple, e.g. 96, wells), especially for cell culture and for microarrays for high-throughput techniques. Other suitable forms for the support of the present invention are coverslips, fibers, foams, particles, membranes, porous scaffolds, meshes or implants.

The nanostructured TiO₂ (ns-TiO₂) film according to the invention consists of TiO₂ nanoparticles (i.e. crystallites) with a diameter below 20 nm, embedded in an amorphous matrix (i.e. of TiO₂) with a density below 75% of bulk TiO₂ density. The ns-TiO₂ film can be formed on the substrate material by deposition of nanoparticles from the gas phase onto the substrate, preferably by means of supersonic cluster beam deposition (SCBD) using the apparatus disclosed in US 6,392,188.

Thus, the present invention further relates to a method for the production of the solid support as defined above, which comprises the steps of:
(a) formation of a nanostructured TiO₂ film at least on areas of the substrate material coming into contact with biological material, i.e. virus and/or cells, by deposition of nanoparticles from the gas-phase onto the substrate, for instance by means of supersonic cluster beam deposition (SCBD) using a pulsed microplasma cluster source; and
(b) contacting the surface of the nanostructured TiO₂ film with viruses and/or cells.

Briefly, the SCBD technique consists in the assembling of clusters produced in supersonic expansions. The clusters are aerodynamically accelerated to hyperthermal energies in order to provide an impact energy high enough to create links between the cluster and the growing material, but not such to destroy the structure of the impinging particle. The production process utilizes a cluster source known as Pulsed Microplasma Cluster Source (PMCS). The process allows the deposition of nanostructured thin films with a precise control on cluster mass distribution and kinetic energy. The PMCS technology consists in the generation of clusters by condensation of plasma of the desired material (i.e. TiO₂) with an inert carrier gas. The process can be carried out with the substrate kept at room temperature. Further details of the deposition apparatus and process are provided in US 6,392,188, which is herein incorporated by reference.

The ns-TiO₂ film deposition process can be set to produce either completely or partially coated substrate materials; generally, the ns-TiO₂ film is deposited on the support surfaces which come into contact with the biological material, i.e. viruses and/or cells.

The term "immobilisation" as used herein means that the viruses and/or cells are attached to the surface of the nanostructured TiO₂ film by any chemical (e.g. by using binding partners such as streptavidin/biotin, antigen/antibody etc.) or physical means (e.g. adhesion or adsorption).

Thus, in a further aspect, the invention provides the use of a nanostructured TiO₂ film, preferably obtained by means of supersonic cluster beam deposition using a pulsed microplasma cluster source, as a substrate for virus adsorption or cell adhesion.

An ultraviolet photoelectron spectroscopy (UPS) analysis shows that the valence band of as deposited ns-TiO₂ films is characterized by states with energies between 3 and 9 eV with respect to the Fermi level (E_{F}). In this range, the peak at about 6 eV and the peak at 8 eV correspond to π (nonbonding) and σ (bonding) O 2p orbital. A considerable presence of gap states at 0.8 eV below the E_{F} is observed. These states are related to Ti³⁺ point defects due to oxygen vacancies. The large porosity and the presence of chemisorption sites in ns-TiO₂ films suggest that the attachment of proteins, the adsorption of viruses and the adhesion of cells may be favoured by the presence of positive electric charge distributed on the surface and by the large active surface area.

Cells or viruses can be adhered to or adsorbed on the surface of TiO₂ films by simple contact of cell preparations (e.g. suspensions) or virus-containing solutions.

According to an alternative embodiment of the invention, streptavidin, avidin or neutravidin is immobilized on the ns-TiO₂ film deposited on a suitable support in order to interact with biotinylated viruses for their attachment on the ns-TiO₂ film.

The present invention further relates to the use of the solid supports according to the invention for infection of cells with viruses, in particular for virus-mediated gene delivery to cells.

Therefore, the present invention generally provides a method for cell infection with viruses *in vitro* comprising the steps of:
(a) providing a solid support of a biocompatible substrate material being at least partially coated with a nanostructured TiO₂ film; and
(b) culturing cells on the nanostructured TiO₂ film-coated support in the presence of an infecting virus.

The method for cell infection according to the invention can thus be carried out by simply culturing the cells on the nanostructured TiO₂ film-coated support in the presence of an infecting virus, i.e. without utilizing a binding pair such as biotin/streptavidin. Thanks to the peculiar characteristics of ns-TiO₂ films, in fact, viruses adhere to the substrate surface and infect the cells as efficiently as with infection enhancers such as polybrene or other polycations; unlike polybrene and polycations, however, ns-TiO₂ coated supports do not cause toxicity problems nor affect cell functionality [11].

According to a preferred embodiment, the infection method comprises the steps of:
(a) providing a solid support of a biocompatible substrate material being at least partially coated with a nanostructured TiO₂ film;
(b) contacting the nanostructured TiO₂ film-coated support with viruses;
(c) contacting the virus adhered on the surface of the nanostructured TiO₂ film-coated support with a cell preparation; and
(d) culturing the cells for a time sufficient for the infection to occur.

Alternatively, the infection method comprises the steps of:
(a) providing a solid support of a biocompatible substrate material being at least partially coated with a nanostructured TiO₂ film;
(b) immobilising streptavidin, avidin or neutravidin on the nanostructured TiO₂ film coating;
(c) contacting the nanostructured TiO₂ film-coated support with a biotinylated virus so as to form a complex of biotinylated virus with the immobilised streptavidin, avidin or neutravidin;
(d) contacting the complex with a cell preparation; and
(e) culturing the cells for a time sufficient for the infection to occur.

The production of biotinylated viruses, e.g. retroviruses can be carried out according to methods known in the art, e.g. as described in [18].

Alternatively, the infection method of the invention comprises the steps of:
(a) providing a solid support of a biocompatible substrate material being at least partially coated with a nanostructured TiO₂ film;
(b) adding a cell preparation to the nanostructured TiO₂ film-coated support;
(c) culturing the cells for an appropriate period of time;
(d) adding a viral supernatant; and
(e) culturing the cells for a time sufficient for the infection to occur.

Preferred viruses for use in the present invention are retroviruses, adenoviruses, adeno-associated viruses (AAV) and any other viruses that can be utilized as vectors for genetic manipulation of cells. "Cells" according to the present invention comprise prokaryotic cells such as bacteria as well as eukaryotic cells such as yeast, plant cells, animal cells, preferably mammalian cells, especially human cells.

In general, any methodology suitable for virus-mediated gene delivery to cells can be carried out using ns-TiO₂ film-coated supports according to the invention. Virus immobilisation on ns-TiO₂ films can be obtained, for example, by means of an anchor molecule such as retronectin, a chimeric peptide of human fibronectin which, when coated on the surface of a suitable support (e.g. petri dishes or flasks), significantly enhances retrovirus-mediated gene transduction into cells [12]. Alternatively, viruses or cells can be genetically modified so as to expose on their surfaces an antigen or binding peptide which is recognized and bound by an antibody or protein immobilised on the ns-TiO₂ film.

In a preferred embodiment, ns-TiO₂ film-coated supports according to the invention are used to set up microarray systems. Therefore, the present invention also provides a microarray device which comprises a solid support of the invention wherein the nanostructured TiO₂ film is in the form of a micro- or nano-pattern. The extremely high collimation obtainable with the SCBD technique allows in fact the production of micro and nano-patterned ns-TiO₂ films with a very high resolution [13, 14]. The micro- or nano-patterned films can be differentially functionalised depending on the desired application. For example, supports coated with microarray-patterned ns-TiO₂ films can be used in genetic and phenotypic assays. For these applications, viruses carrying different genetic inserts are spotted on the microarray and used to infect cells. Infected cells are then analysed for the integration or expression of the exogenous genetic material using suitable detection systems.

In addition, the ns-TiO₂ coated materials according to the invention can be used to develop methods for gene therapy and cell replacement therapy, e.g. systems to perform localized infection through TiO₂ nanoparticles loaded with viruses that can be implanted in specific tissues to favour high levels of local gene transduction. The ns-TiO₂ coated materials according to the invention are also useful for *ex vivo* gene therapy.

Therefore, the present invention provides implantable particles or devices, i.e. any two or three dimensional body (e.g. a chip) of a nanostructured. TiO₂ film-coated biocompatible material loaded with viruses. The viruses may be adhered to or adsorbed on the surface of the TiO₂ film-coated material, or may be attached thereto by use of a binding pair or other means as described above.

The invention is further illustrated by the following non-limiting Examples and by the attached Figures.

### Brief description of the Figures

- Fig. 1:: Streptavidin adsorption on ns-TiO₂. Spotting of streptavidin-Cy3 on a layer of ns-TiO₂. Incubation at 37°C in culture medium for different time periods (0, 8, 24 and 48 h).
- Fig. 2A:: Classical infection of melanocytes with retroviral supernatant (GFP).
- Fig. 2B:: 'Reverse infection' of melanocytes with retroviral supernatant (GFP). Comparison of different substrates (plastic, gelatin coated coverslips and ns-TiO₂ in relation to infection efficiency
- Fig. 3:: Retroviral microarray with U20S cells.

### Examples

### 1. Preparation of ns-TiO₂ substrate

Nanostructured TiO₂ films have been deposited by a Supersonic Cluster Beam Deposition (SCBD) apparatus equipped with a Pulsed Microplasma Cluster Source (PMCS) [15]. Briefly, a titanium target is sputtered by a confined plasma jet of an inert gas (He or Ar). Sputtered Ti atoms thermalize within the inert gas and condense to form clusters. The Ti clusters are either oxidized by interaction with residual gas in the background vacuum or by the introduction of a suitable amount of oxygen in the process. The mixture of clusters and inert gas is then extracted in vacuum through a nozzle to form a seeded supersonic beam which is collected on a substrate located in the beam trajectory. The kinetic energy of the clusters is low enough to avoid fragmentation and hence a nanostructured film is grown. The mass distribution of the clusters can be controlled by aerodynamic focusing in order to tailor the nanostructure of the film [16].

### 2. Cell-infection assay on ns-TiO₂ array

Viral vectors are prepared by Ca(PO₄)₂ transfection procedures in Amphotropic Phoenix packaging cells [17]. Cells are biotinylated *in vivo* [18] and viral supernatant is collected, concentrated 10 times with 8% PEG8000 after overnight incubation at 4°C and aliquoted in presence of 100 µg/ml of a stabilizing sugar, preferably trehalose, at -80°C.

Viral titration indicates different viral concentration ranging from 10⁸ to 10¹² cfu/ml.

A monolayer of protein (streptavidin) ranging between 1 µg/ml to 0.1 µg/ml in Hepes 10 mM/NaCl 150 mM buffer is prepared on the nanostructured TiO₂ slide by robotic spotting, incubated to allow adsorption, and the monolayer is stabilized by a treatment with 10% serum. Biotinylated virus is then deposited by robotic spotting on the functionalized substrate: after an incubation time to allow virus binding, wash steps eliminate the viral excess and cells are plated on the substrate.

To perform analysis of the infected array after 48-72 hours cells are processed for immunodetection by microscopy or treated with the appropriate antibiotic (puromicine, hygromicine, G418) to perform selection and obtain a homogeneous population of cells growing in clusters, expressing or down-regulating at high efficiency the gene of interest. At the end of selection the slide is processed for microscopy or scanner detection.

### 3. Streptavidin adsorption on ns-TiO₂

0.1 µg/ml streptavidin labelled with Cy3 in 150mM NaCl, 10 mM Hepes was spotted on a slide coated with nanostructured TiO₂ film.

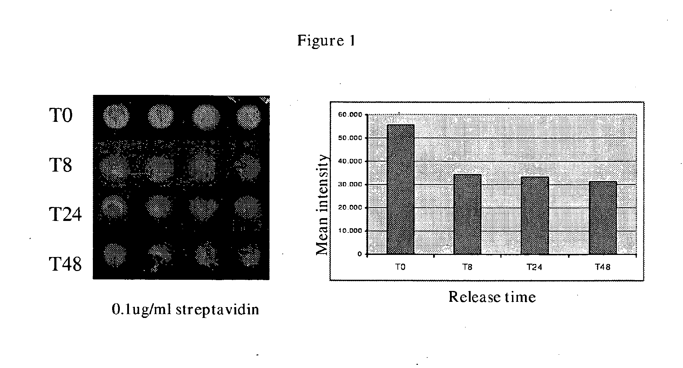

The slide was incubated in saline medium at 37°C for different time points (0, 8, 24, 48hr) to verify whether the spotted protein had been stably adsorbed onto the TiO₂ surface. Afterwards, the slide was scanned to determine the fluorescence intensity. The results (Figure 1) show that after 8 hrs the fluorescence intensity is constant, indicating that streptavidin molecules form a stable layer adsorbed on TiO₂.

### 4. ns-TiO₂-mediated melanocyte infection in the absence of polybrene

Primary melanocytes were used as target cells. Briefly, for the classical infection protocols cells were plated on a plastic support (control) and on ns-TiO₂ coated coverslips. After 24 hours, the cells were infected for 12 hrs with a GFP-expressing virus in solution in the presence or absence of polybrene. 72 hrs later, the cells were fixed with 4% paraformaldehyde for 10 minutes and the nuclei were stained with DAPI.

Cells were analysed with a fluorescence microscope. The infection efficiency and the mean fluorescence intensity were calculated for each sample using an image analysis software.

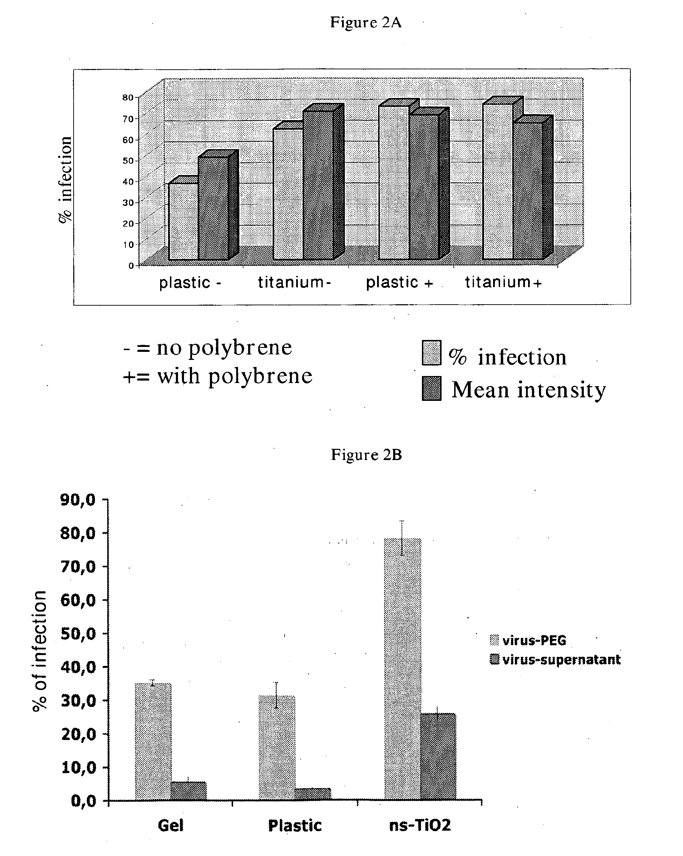

The results (Figure 2A) show that the infections via ns-TiO₂ in the absence of polybrene and, on the plastic support in the presence of polybrene, respectively, have the same efficiency and mean intensity.

For the "reverse infection" protocol, different substrates were compared for infection efficiency: briefly, a ns-TiO₂ coated coverslip, a gelatin-coated coverslip and a plastic well were incubated with viral preparation (GFP-expressing virus) for 4 hours at 4°C (Virus-PEG correspond to a 10 fold concentrated viral preparation, Virus-supernatant correspond to the not concentrated viral preparation).

After a brief wash with PBS, melanocytes were plated on all the samples. After 72 hours cells were analysed with a fluorescence microscope. The infection efficiency and the mean fluorescence intensity were calculated for each sample using an image analysis software.

The results (Figure 2B) show that the infections mediated by ns-TiO₂ in the absence of polybrene, are more efficient compared to others substrates (gelatin and plastic).

### 5. Retroviral microarray with U20S cells

A slide was coated with an ns-TiO₂ film using supersonic cluster beam deposition. The slide was spotted with streptavidin, incubated and washed to eliminate the protein excess; subsequently, the biotinylated virus was spotted in the corresponding spots. Two different virus encoding fluorescent proteins locating at different cell-compartments - and staining the whole cell and the nucleolar dots, respectively - were used. This system allows the identification of the cell clusters specifically expressing the different viruses.

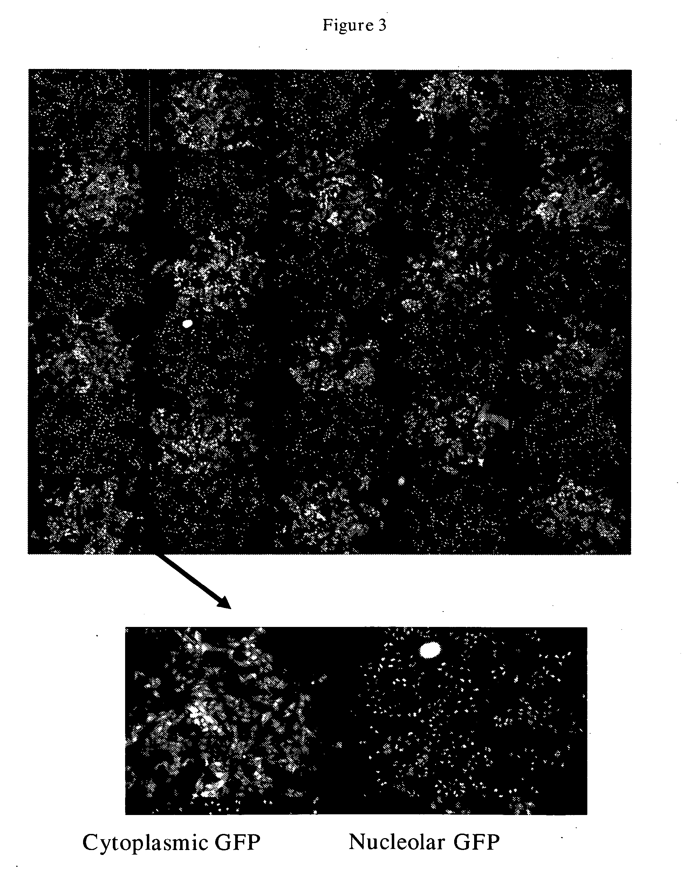

The slide was incubated to allow streptavidin/virus binding, washed and thereafter the cells were plated. After a period of 72 hours, the slide was fixed with 4% paraformaldehyde for 10 min and the cell nuclei were stained with DAPI. Image acquisition and analysis was carried out with an automated microscope.
The results are illustrated in Figure 3.

### REFERENCES

1. Stoll D et al., Drug Discovery Today: Targets 3, 24 (2004).
2. Jordan M, Wurm F, Methods 33, 136 (2004).
3. Wells DJ, Gene Therapy 11, 1363 (2004).
4. Chou TH, Biswas S, Lu S, Methods Mol. Biol. 245, 147 (2004).
5. Hansen AC, Pedersen FS, Curr. Opin. Mol. Ther. 4, 324 (2002).
6. Bruneel N, Helsen JA, J. Biomed. Mater. Res. 22, 203 (1988).
7. Hook F et al., Colloids Surf. B: Biointerfaces 24, 155 (2002).
8. Gutwein LG, Webster TJ, J. Nanopart. Res. 4, 231 (2002).
9. Kasemo B, Surf. Sci. 500, 656 (2002).
10. Paunesku T et al., Nature Mater. 2, 343 (2003).
11. Cornetta K, Anderson WF, J. Virol. Methods 23, 187 (1989).
12. Hanenberg H. et al., Nature Med. 2, 876 (1996).
13. Barborini E et al., Appl. Phys. Lett. 81, 3052 (2002).
14. Barborini E et al., Appl. Phys. Lett. 77, 1059 (2000).
15. Barborini E et al., J. Phys. D 32, L105 (1999).
16. Piseri P et al., Rev. Sci. Instrum. 72, 2261 (2001).
17. Kinsella TM et al., Hum. Gene Ther. 7, 1405 (1996).
18. Hughes C et al., Molecular Therapy 3, 623 (2001).
19. Topoglidis E et al., Anal. Chem. 70, 5111 (1998).

## Claims

1. A solid support of a biocompatible substrate material being at least partially coated with a nanostructured TiO₂ film having viruses and/or cells immobilised on the surface thereof wherein the film of nanostructured TiO₂ consists of TiO₂ nanoparticles with a diameter below 20 nm embedded in an amorphous TiO₂ matrix with a density of below 75% of bulk TiO₂ density.

2. The solid support of claim 1 being in the form of a slide, dish, flask, plate, coverslip, fiber, foam, particle, membrane, porous scaffold, mesh or implant.

3. The solid support of claim 1 or 2 wherein the substrate is glass, plastic, ceramic, metal or a biodegradable or undegradable biopolymeric material.

4. The solid support according to any one of claims 1 to 3 wherein biotinylated viruses are immobilised on the nanostructured TiO₂ film carrying streptavidin, avidin or neutravidin.

5. A microarray device comprising the solid support according to any one of claims 1 to 4 wherein the nanostructured TiO₂ film is in the form of a micro- or nano-pattern.

6. The microarray device of claim 5 wherein viruses carrying different genetic inserts are spotted on the surface of the nanostructured TiO₂ film.

7. A method for the production of the solid support according to any one of claims 1 to 4 comprising the steps of:
(a) depositing a nanostructured TiO₂ film at least on areas of the substrate material coming into contact with viruses and/or cells by means of nanoparticle deposition from the gas-phase; and
(b) contacting the surface of the nanostructured TiO₂ film with viruses and/or cells.

8. The method of claim 7 wherein the nanoparticle deposition from the gas-phase is carried out by means of supersonic cluster beam deposition (SCBD) using a pulsed micro plasma cluster source.

9. Use of the solid support according to any one of claims 1 to 4 for virus-mediated gene delivery to cells on the solid support.

10. A method for cell infection with viruses *in vitro* comprising the steps of:
(a) providing a solid support of a biocompatible substrate material being at least partially coated with a nanostructured TiO₂ film consisting of TiO₂ nanoparticles with a diameter below 20 nm embedded in an amorphous TiO₂ matrix with a density of below 75% of bulk TiO₂ density; and
(b) culturing cells on the nanostructured TiO₂ film-coated support in the presence of an infecting virus.

11. The method of claim 10 comprising the steps of:
(a) providing a solid support of a biocompatible substrate material being at least partially coated with a nanostructured TiO₂ film consisting of TiO₂ nanoparticles with a diameter below 20 nm embedded in an amorphous TiO₂ matrix with a density of below 75% of bulk TiO₂ density;
(b) contacting the nanostructured TiO₂ film-coated support with viruses;
(c) contacting the virus adhered on the surface of the nanostructured TiO₂ film-coated support with a cell preparation; and
(d) culturing the cells for a time sufficient for the infection to occur.

12. The method of claim 10 comprising the steps of:
(a) providing a solid support of a biocompatible substrate material being at least partially coated with a nanostructured TiO₂ film consisting of TiO₂ nanoparticles with a diameter below 20 nm embedded in an amorphous TiO₂ matrix with a density of below 75% of bulk TiO₂ density;
(b) immobilising streptavidin, avidin or neutravidin on the nanostructured TiO₂ film coating;
(c) contacting the nanostructured TiO₂ film-coated support with a biotinylated virus so as to form a complex of biotinylated viruses with the immobilised streptavidin, avidin or neutravidin;
(d) contacting the complex with a cell preparation; and
(e) culturing the cells for a time sufficient for the infection to occur.

13. The method of claim 10 comprising the steps of:
(a) providing a solid support of a biocompatible substrate material being at least partially coated with a nanostructured TiO₂ film consisting of TiO₂ nanoparticles with a diameter below 20 nm embedded in an amorphous TiO₂ matrix with a density of below 75% of bulk TiO₂ density;
(b) adding a cell preparation to the nanostructured TiO₂ film-coated support;
(c) culturing the cells for an appropriate period of time;
(d) adding a viral supernatant;
(e) culturing the cells for a time sufficient for the infection to occur.

14. The method according to any one of claims 10 to 13 wherein the viruses are retroviruses, adenoviruses, adeno-associated viruses (AAV) and any other viruses that can be utilized as vectors for genetic manipulation of cells.

15. The method according to any one of claims 10 to 14 wherein the viruses are genetically modified.

16. Implantable particles or device of a nanostructured TiO₂ film-coated biocompatible material loaded with viruses, wherein the film of nanostructured TiO₂ consists of TiO₂ nanoparticles with a diameter below 20 nm embedded in an amorphous TiO₂ matrix with a density of below 75% of bulk TiO₂ density.

## Patentansprüche

1. Fester Träger aus einem biokompatiblen Substratmaterial, das mindestens teilweise mit einem nanostrukturierten TiO₂-Film beschichtet ist, der auf seiner Oberfläche immobilisierte Viren und/oder Zellen aufweist, wobei der Film aus nanostrukturiertem TiO₂ aus in eine amorphe TiO₂-Matrix mit einer Dichte von weniger als 75% der TiO₂-Rohdichte eingebetteten TiO₂-Nanopartikeln mit einem Durchmesser von weniger als 20 nm besteht.

2. Fester Träger nach Anspruch 1 in Form eines Objektträgers, einer Schale, einer Flasche, einer Platte, eines Deckgläschens, einer Faser, eines Schaumstoffs, eines Partikels, einer Membran, eines porösen Gerüsts, eines Netzes oder eines Implantats.

3. Fester Träger nach Anspruch 1 oder 2, wobei das Substrat Glas, Plastik, Keramik, Metall oder ein biologisch abbaubares oder nicht abbaubares biopolymeres Material ist.

4. Fester Träger nach einem der Ansprüche 1 bis 3, wobei biotinylierte Viren auf dem nanostrukturierten TiO₂-Film, der Streptavidin, Avidin oder Neutravidin enthält, immobilisiert sind.

5. Microarray-Vorrichtung, die den festen Träger nach einem der Ansprüche 1 bis 4 umfasst, wobei der nanostrukturierte TiO₂-Film in Form eines Mikro- oder Nano-Musters vorliegt.

6. Microarray-Vorrichtung nach Anspruch 5, wobei Viren, die unterschiedliche genetische Insertionen aufweisen, auf die Oberfläche des nanostruktierten Films aufgebracht werden.

7. Verfahren zur Herstellung eines festen Trägers nach einem der Ansprüche 1 bis 4, umfassend die Schritte:
(a) Aufbringen eines nanostrukturierten TiO₂-Films mindestens auf die Bereiche des Substratmaterials, die mit Viren und/oder Zellen in Kontakt kommen, mittels Nanopartikel-Abscheidung aus der Gas-Phase; und
(b) Inkontaktbringen der Oberfläche des nanostrukturierten TiO₂-Films mit Viren und/oder Zellen.

8. Verfahren nach Anspruch 7, wobei die Nanopartikel-Abscheidung aus der Gasphase mittels Ultraschall-Teilchenstrahl-Abscheidung (supersonic cluster beam deposition, SCBD) mit einer gepulsten Mikroplasma-Teilchenquelle durchgeführt wird.

9. Verwendung des festen Trägers nach einem der Ansprüche 1 bis 4 zum Viren-vermittelten Gentransfer in Zellen auf dem festen Träger.

10. Verfahren zur *In-vitro*-Infektion von Zellen mit Viren, umfassend die Schritte:
(a) Bereitstellen eines festen Trägers aus einem biokompatiblen Substratmaterial, das mindestens teilweise mit einem nanostrukturierten TiO₂-Film beschichtet ist, der aus in eine amorphe TiO₂-Matrix mit einer Dichte von weniger als 75% der TiO₂-Rohdichte eingebettenen TiO₂-Nanopartikeln mit einem Durchmesser von weniger als 20 nm besteht; und
(b) Züchten von Zellen auf dem mit einem nanostrukturierten TiO₂-Film beschichteten Träger in Gegenwart eines infizierenden Virus.

11. Verfahren nach Anspruch 10, umfassend die Schritte:
(a) Bereitstellen eines festen Trägers aus einem biokompatiblen Substratmaterial, das mindestens teilweise mit einem nanostrukturierten TiO₂-Film beschichtet ist, der aus in eine amorphe TiO₂-Matrix mit einer Dichte von weniger als 75% der TiO₂-Rohdichte eingebettenen TiO₂-Nanopartikeln mit einem Durchmesser von weniger als 20 nm besteht;
(b) Inkontaktbringen des mit einem nanostrukturierten TiO₂-Film beschichteten Trägers mit Viren;
(c) Inkontaktbringen des Virus, der an der Oberfläche des mit einem nanostrukturierten TiO₂-Film beschichteten Trägers haftet, mit einer Zellpräparation; und
(d) Züchten der Zellen für eine Zeitspanne, die ausreicht, um eine Infektion herbeizuführen.

12. Verfahren nach Anspruch 10, umfassend die Schritte:
(a) Bereitstellen eines festen Trägers aus einem biokompatiblen Substratmaterial, das mindestens teilweise mit einem nanostrukturierten TiO₂-Film beschichtet ist, der aus in eine amorphe TiO₂-Matrix mit einer Dichte von weniger als 75% der TiO₂-Rohdichte eingebettenen TiO₂-Nanopartikeln mit einem Durchmesser von weniger als 20 nm besteht;
(b) Immobilisieren von Streptavidin, Avidin oder Neutravidin auf der Beschichtung aus dem nanostrukturierten TiO₂-Film;
(c) Inkontaktbringen des mit einem nanostrukturierten TiO₂-Film beschichteten Trägers mit einem biotinylierten Virus derart, dass ein Komplex aus biotinylierten Viren mit dem immobilisierten Streptavidin, Avidin oder Neutravidin entsteht;
(d) Inkontaktbringen des Komplexes mit einer Zellpräparation; und
(e) Züchten der Zellen für eine Zeitspanne, die ausreicht, um eine Infektion herbeizuführen.

13. Verfahren nach Anspruch 10, umfassend die Schritte:
(a) Bereitstellen eines festen Trägers aus einem biokompatiblen Substratmaterial, das mindestens teilweise mit einem nanostrukturierten TiO₂-Film beschichtet ist, der aus in eine amorphe TiO₂-Matrix mit einer Dichte von weniger als 75% der TiO₂-Rohdichte eingebettenen TiO₂-Nanopartikeln mit einem Durchmesser von weniger als 20 nm besteht
(b) Hinzugeben einer Zellpräparation zu dem mit einem nanostrukturierten TiO₂-Film beschichteten Träger;
(c) Züchten der Zellen für eine geeignete Zeitspanne;
(d) Hinzugeben eines viralen Überstands;
(e) Züchten der Zellen für eine Zeitspanne, die ausreicht, um eine Infektion herbeizuführen.

14. Verfahren nach einem der Ansprüche 10 bis 13, wobei die Viren Retroviren, Adenoviren, Adeno-assoziierte Viren (AAV) oder beliebige andere Viren sind, die als Vektoren zur genetischen Manipulation von Zellen eingesetzt werden können.

15. Verfahren nach einem der Ansprüche 10 bis 14, wobei die Viren genetisch modifiziert sind.

16. Mit Viren beladene implantierbare Partikel oder Vorrichtung aus einem mit einem nanostrukturierten TiO₂-Film beschichteten biokompatiblen Material, wobei der nanostrukturierte TiO₂-Film aus in eine amorphe TiO₂-Matrix mit einer Dichte von weniger als 75% der TiO₂-Rohdichte eingebetteten nanostrukturierten TiO₂-Partikeln mit einem Durchmesser von weniger als 20 nm besteht.

## Revendications

1. Support solide d'un matériau substrat biocompatible étant au moins partiellement revêtu d'un film de TiO₂ nanostructuré ayant des virus et/ou des cellules immobilisés sur sa surface où le film de TiO₂ nanostructuré est constitué de nanoparticules de TiO₂ avec un diamètre inférieur à 20 nm incorporées dans une matrice de TiO₂ amorphe avec une densité inférieure à 75 % de la densité du TiO₂ à l'état brut.

2. Support solide selon la revendication 1 étant sous la forme d'une lame, d'une boîte, d'un ballon, d'une plaque, d'une lamelle, d'une fibre, d'une mousse, d'une particule, d'une membrane, d'une matrice de support poreuse, d'une maille ou d'un implant.

3. Support solide selon la revendication 1 ou 2 où le substrat est du verre, du plastique, de la céramique, du métal ou un matériau biopolymère biodégradable ou non dégradable.

4. Support solide selon l'une quelconque des revendications 1 à 3 où des virus biotinylés sont immobilisés sur le film de TiO₂ nanostructuré portant de la streptavidine, de l'avidine ou de la neutravidine.

5. Dispositif de micropuce comprenant le support solide selon l'une quelconque des revendications 1 à 4 où le film de TiO₂ nanostructuré est sous la forme d'une microstructure ou d'une nanostructure.

6. Dispositif de micropuce selon la revendication 5 où des virus portant des inserts génétiques différents sont déposés sur la surface du film de TiO₂ nanostructuré.

7. Procédé de production du support solide selon l'une quelconque des revendications 1 à 4 comprenant les étapes consistant à :
(a) déposer un film de TiO₂ nanostructuré au moins sur des zones du matériau substrat entrant en contact avec des virus et/ou des cellules au moyen du dépôt de nanoparticules issues de la phase gazeuse ; et
(b) mettre en contact la surface du film de TiO₂ nanostructuré avec des virus et/ou des cellules.

8. Procédé selon la revendication 7 dans lequel le dépôt de nanoparticules issues de la phase gazeuse est réalisé au moyen d'un dépôt supersonique de faisceaux d'agrégats (SCBD) en utilisant une source d'agrégats microplasmatiques pulsés.

9. Utilisation du support solide selon l'une quelconque des revendications 1 à 4 pour délivrer au moyen de virus du matériel génétique à des cellules sur le support solide.

10. Procédé d'infection cellulaire avec des virus *in vitro* comprenant les étapes consistant à :
(a) fournir un support solide d'un matériau substrat biocompatible étant au moins partiellement revêtu d'un film de TiO₂ nanostructuré constitué de nanoparticules de TiO₂ avec un diamètre inférieur à 20 nm incorporées dans une matrice de TiO₂ amorphe avec une densité inférieure à 75 % de la densité du TiO₂ à l'état brut ; et
(b) cultiver les cellules sur le support revêtu du film de TiO₂ nanostructuré en présence d'un virus infectieux.

11. Procédé selon la revendication 10 comprenant les étapes consistant à :
(a) fournir un support solide d'un matériau substrat biocompatible étant au moins partiellement revêtu d'un film de TiO₂ nanostructuré constitué de nanoparticules de TiO₂ avec un diamètre inférieur à 20 nm incorporées dans une matrice de TiO₂ amorphe avec une densité inférieure à 75 % de la densité du TiO₂ à l'état brut ;
(b) mettre en contact le support revêtu du film de TiO₂ nanostructuré avec des virus ;
(c) mettre en contact le virus qui a adhéré à la surface du support revêtu du film de TiO₂ nanostructuré avec une préparation cellulaire ; et
(d) cultiver les cellules pendant une durée suffisante pour que l'infection se produise.

12. Procédé selon la revendication 10 comprenant les étapes consistant à :
(a) fournir un support solide d'un matériau substrat biocompatible étant au moins partiellement revêtu d'un film de TiO₂ nanostructuré constitué de nanoparticules de TiO₂ avec un diamètre inférieur à 20 nm incorporées dans une matrice de TiO₂ amorphe avec une densité inférieure à 75 % de la densité du TiO₂ à l'état brut ;
(b) immobiliser de la streptavidine, de l'avidine ou de la neutravidine sur le revêtement à base de film de TiO₂ nanostructuré ;
(c) mettre en contact le support revêtu du film de TiO₂ nanostructuré avec un virus biotinylé afin de former un complexe de virus biotinylés avec la streptavidine, l'avidine ou la neutravidine immobilisée ;
(d) mettre en contact le complexe avec une préparation cellulaire ; et
(e) cultiver des cellules pendant une durée suffisante pour que l'infection se produise.

13. Procédé selon la revendication 10 comprenant les étapes consistant à :
(a) fournir un support solide d'un matériau substrat biocompatible étant au moins partiellement revêtu d'un film de TiO₂ nanostructuré constitué de nanoparticules de TiO₂ avec un diamètre inférieur à 20 nm incorporées dans une matrice de TiO₂ amorphe avec une densité inférieure à 75 % de la densité du TiO₂ à l'état brut ;
(b) ajouter une préparation cellulaire au support revêtu du film de TiO₂ nanostructuré ;
(c) cultiver les cellules pendant une période de temps appropriée ;
(d) ajouter un surnageant viral ;
(e) cultiver les cellules pendant une durée suffisante pour que l'infection se produise.

14. Procédé selon l'une quelconque des revendications 10 à 13 dans lequel les virus sont des rétrovirus, des adénovirus, des virus adéno-associés (AAV) et tous les autres virus qui peuvent être utilisés en tant que vecteurs pour une manipulation génétique des cellules.

15. Procédé selon l'une quelconque des revendications 10 à 14 dans lequel les virus sont génétiquement modifiés.

16. Particules implantables ou dispositif d'un matériau biocompatible revêtu d'un film de TiO₂ nanostructuré chargé avec des virus, où le film de TiO₂ nanostructuré est constitué de nanoparticules de TiO₂ avec un diamètre inférieur à 20 nm incorporées dans une matrice de TiO₂ amorphe avec une densité inférieure à 75 % de la densité du TiO₂ à l'état brut.
